(19) Europäisches Patentamt

European Patent Office

Office européen des brevets

(11) Numéro de publication:

# 0 092 015
## A1

⑫ **DEMANDE DE BREVET EUROPEEN**

㉑ Numéro de dépôt: **82810160.0**

㉒ Date de dépôt: **16.04.82**

(51) Int. Cl.³: **A 61 N 1/30, A 61 N 1/08**

㊸ Date de publication de la demande: **26.10.83**
**Bulletin 83/43**

㊤ Etats contractants désignés: **AT BE CH DE FR GB IT LI LU NL SE**

⑪ Demandeur: **Brodard, Roland, Avenue des Châtaigniers, 4, CH-1844 Villeneuve (CH)**

㋐ Inventeur: **Brodard, Roland, Avenue des Châtaigniers, 4, CH-1844 Villeneuve (CH)**

㊞ Mandataire: **Steiner, Martin et al, c/o AMMANN INGENIEURS-CONSEILS EN PROPRIETE INTELLECTUELLE SA BERNE Schwarztorstrasse 31, CH-3001 Bern (CH)**

㊹ **Dispositif d'ionisation.**

㊗ Le dispositif comprend un générateur de courant (3, 6) réglable par un potentiomètre (2). Une résistance (11) est connectée en série avec une paire d'électrodes amovibles (10a, 10b) dans un circuit de contre-réaction du générateur par l'intermédiaire d'un transistor (8). La résistance (11) est solidaire des électrodes et détermine l'intensité du courant dans celles-ci. Sa valeur est déterminée par la surface des électrodes pour maintenir constante la densité de courant. Une diode LED (14) associée à un circuit (13) donne une indication de la densité de courant dans les électrodes. Une diode LED (19) associée à un comparateur (16, 17, 18) signale une saturation du transistor (8) limitant le courant lorsque la résistance entre les électrodes dépasse une valeur critique. Un comparateur (21) commandant une diode LED (22) de signalisation de sécurité et un second transistor (24) permettent d'interrompre le courant dans les électrodes si, en raison d'une forte résistance entre ces dernières, la tension sur la résistance (11) tombe en dessous de la tension d'entrée du générateur.

1

## DISPOSITIF D'IONISATION

La présente invention concerne un dispositif d'ionisation comportant au moins un générateur de courant réglable et au moins une paire d'électrodes branchées de manière amovible audit générateur par des moyens de connexion.

Il devient de plus en plus fréquent, pour certains traitements dans le domaine de l'esthétique ou de la rhumatologie, d'utiliser le procédé d'ionisation. Cette ionisation consiste à faire passer un courant continu de faible intensité entre deux parties du corps du patient, ce qui provoque une électrolyse facilitant la pénétration de la solution de traitement. Les appareils utilisés à cet effet comportent plusieurs paires d'électrodes enfichables reliées chacune à un générateur de courant réglable. Ces générateurs sont isolés entre eux et comportent généralement un milliampèremètre permettant de mesurer le courant dans la sonde correspondante.

Il est en effet de la plus grande importance de doser avec exactitude ce courant si l'on veut éviter les risques de brûlures, brûlures d'autant plus dangereuses qu'elles peuvent être indolores au moment de leur formation. Or, l'intensité du courant de consigne varie d'une paire d'électrodes à l'autre car, comme pour toute électrolyse, c'est l'intensité de courant par unité de surface qui importe, et l'on doit donc tenir compte des dimensions de ces électrodes. Pour celà, on utilise généralement une table de conversion, mais certains fabricants graduent directement les instruments en $mA/cm^2$, ce qui nécessite plusieurs échelles correspondant chacune à un type d'électrodes de surface déterminée. Ces moyens sont limitatifs et il n'excluent en aucun cas les erreurs de manipulation, erreurs qui peuvent avoir des conséquences fâcheuses pour le patient.

Le but de la présente invention est de remédier aux désavantages mentionnés ci-dessus, c'est-à-dire de réaliser un dispositif d'ionisation supprimant pratiquement et automatiquement tout risque de brûlures dû à des erreurs de manipulation.

Pour atteindre ce but, le dispositif selon l'invention comprend en outre des moyens reliés audit générateur pour déterminer l'intensité du courant délivré par ledit générateur auxdites électrodes, lesdits moyens dépendant du type de la paire d'électrodes branchée audit générateur.

Par le fait que les moyens dépendent de la paire d'électrodes connectée au générateur, le courant de ce dernier, et plus précisement la densité de courant entre les électrodes, peut être déterminé automatiquement de manière à éliminer tout risque de brûlures du patient.

L'invention va être décrite ci-après, à titre d'exemple et à l'aide du dessins dans lequel:

La fig. 1 représente schématiquement le circuit du dispositif selon l'invention, comportant un générateur de courant simplifié,

La fig. 2 représente le circuit de la fig. 1 complété par des moyens de sécurité et de contrôle, et

La fig. 3 représente un exemple de graduation et de disposition des éléments de contrôle et de réglage.

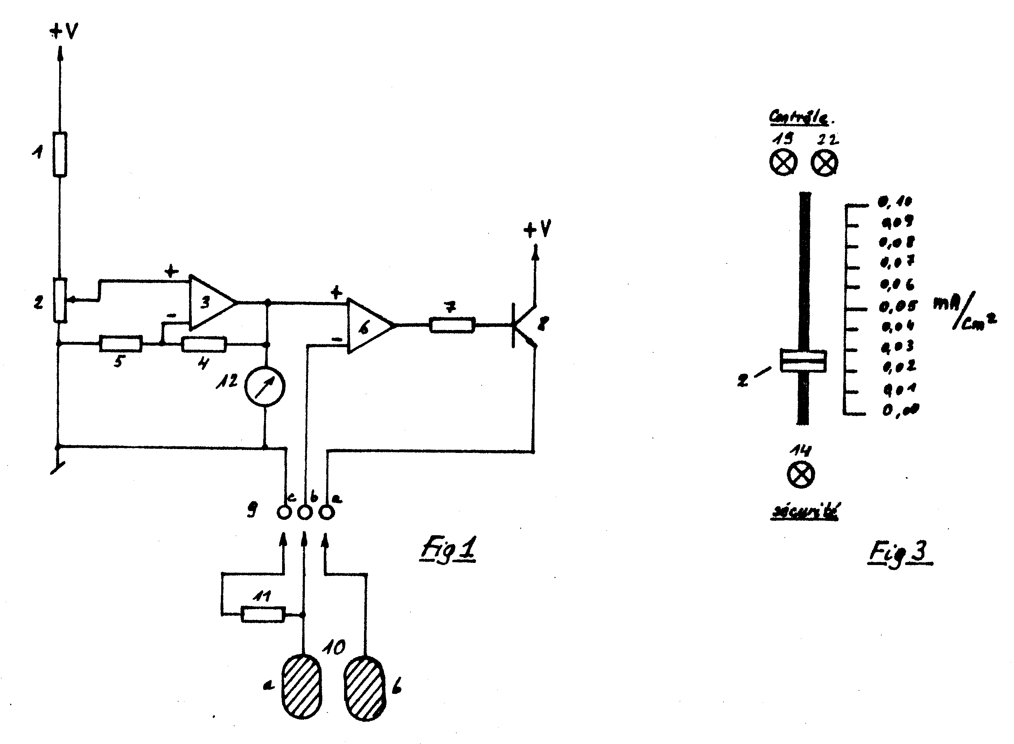

Le générateur de courant de la fig. 1 est formé d'un diviseur de tension réglable formé d'une résistance 1 reliée au pôle positif de l'alimentation et d'un potentiomètre 2 relié au pôle négatif de l'alimentation (masse). Le curseur du potentiomètre est relié à l'entrée directe (+) d'un amplificateur opérationnel 3 dont la sortie est reliée par une résistance 4 à son entrée qui inverse (-), elle-même reliée à la masse par une résistance 5. Cette configuration bien connue permet d'obtenir à la sortie de l'amplificateur une tension proportionelle à la tension UE sur son entrée directe selon la relation

$$US = UE \frac{R4 + R5}{R5} .$$

La tension UE dépend de la position du potentiomètre et varie de 0 à UEmax, de sorte que Us varie de 0 à Usmax. Si le potentiomètre est linéaire, la tension de sortie est donnée par $Us = Usmax \cdot \alpha$ où $\alpha$ représente la position du curseur du potentiomètre par rapport à la course totale.

La tension Us est appliquée à l'entrée directe d'un deuxième amplificateur opérationnel 6 dont la sortie est reliée par une résistance 7 à la base d'un transistor 8 branché en collecteur commun, ce transistor faisant office d'amplificateur de puissance.

L'émetteur du transistor 8 est relié à la borne 9a d'un connecteur 9 à trois pôles dont la borne 9b est reliée à l'entrée qui inverse de l'amplificteur 6, et dont la borne 9c est connectée à la masse. Le connecteur 9 permet de relier le générateur à une paire d'électrodes 10a et 10b.

Les électrodes d'une paire sont généralement de même surface, mais elles peuvent être de surfaces différentes selon les parties du corps du patient auxquelles elles sont appliquées. L'électrode de plus petite surface est alors déterminante pour la densité du courant délivré entre les électrodes. Elles comportent généralement un treillis métallique monté sur caoutchouc et recouvert d'un tissu ou d'une éponge. Chaque électrode est reliée par un conducteur souple à une borne d'une fiche adéquate.

Dans le dispositif selon l'invention, cette fiche comporte au moins une troisième borne c à laquelle est branché un élément de réglage, en l'occurence une résistance 11 reliée par ailleurs à l'électrode 10a. Cette résistance peut être montée directement dans la fiche ou ecore sur l'électrode 10a, mais dans tous les cas elle doit rester solidaire de la paire d'électrodes, l'ensemble électrodes-résistance formant un tout.

Voyons ce qui se passe lorsque la paire d'électrodes est branchée, c'est-à-dire lorsque la fiche est en place sur le connecteur 9. La paire d'électrodes 10 est alors branchée en contre-réaction par la résistance 7, le transistor 8, la borne 9a et la borne 9b entre la sortie et l'entrée qui inverse de l'amplificateur 6, alors que la résistance 11 est reliée par les bornes 9b et 9c entre l'entrée qui inverse de l'amplificateur 6 et la masse.

Dans cette configuratin bien connue, la tension à l'entrée qui inverse de l'amplificateur 6 est maintenue à la même valeur que la tension sur son entrée directe, ceci pour autant qu'un courant suffisant puisse passer entre les électrodes 10a et 10b. C'est le cas lorsque les électrodes ont été mises correctement en place sur le patient. La valeur du courant Ie dans les électrodes est alors déterminée par la tension entre l'entrée qui inverse de l'amplificateur 6 et la masse, selon la relation:

$$Ie = \frac{\alpha \cdot Usmax}{R11}$$

La valeur du courant dépend donc d'une part de la position $\alpha$ du curseur du potentiomètre, ce qui est évident, mais également de la résistance R11. La valeur de cette dernière est choisie en fonction de la surface des électrodes:

$$R11 = \frac{Ro}{S} \qquad S = \text{surface des électrodes} \qquad Ro = \text{constante}$$

le courant devient alors égal à

$$Ie = \frac{\alpha \cdot Usmax}{Ro/S}$$

$$\text{d'où} \quad \frac{Ie}{S} = \frac{\alpha \cdot Usmax}{Ro}$$

On voit donc que par un choix adéquat de la valeur de la résistance 11, le courant par unité de surface, c'est-à-dire la densité de courant devient indépendant du type de la paire d'électrodes utilisée et qu'il ne dépend plus que de la position $\alpha$ du potentiomètre. Ainsi, pour des paires d'électrodes ayant entre elles un rapport de 10 en surface (surface des plus petites électrodes de chaque paire), on aura un rapport de 10 entre les valeurs des résistances R11 associées et aussi entre les intensités de courant, mais on aura une densité de courant uniforme.

Par exemple, pour une électrode de 250 $cm^2$, on choisit Usmax = 2,5V et R11 = 100 ohm, ce qui permet de régler le courant à une valeur maximum de 25 mA correspondant à une densité de courant de 0,1 $mA/cm^2$.

Par contre pour une électrode de 25$cm^2$ et Usmax de 2,5V, on choisit R11 égal à 1 kiloohm, ce qui permet un courant maximum de 2,5 mA correspondant toujours à une densité de courant de 0,1 $mA/cm^2$.

On a ainsi une adaption automatique de l'intensité du courant à la surface des électrodes utilisées, ce qui permet d'éviter tout risque de surintensité et de brûlure.

Il est possible de vérifier cette densité de courant au moyen d'un instrument 12 gradué en $mA/cm^2$. Toutefois, il est également possible de graduer directement le potentiomètre puisque la densité de courant dépend directement de la position de son curseur, du moins lorsque le fonctionnement du dispositif est normal.

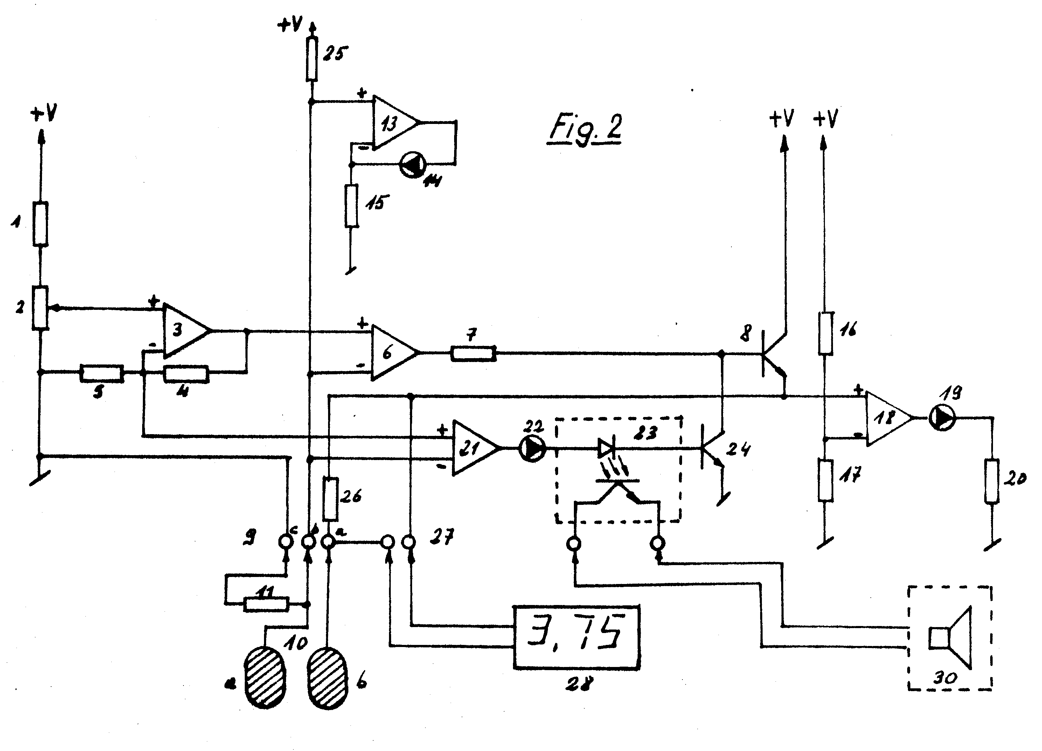

Cependant, il peut se produire certaines anomalies qu'il est souhaitable de signaler, ce qui est prévu dans le schéma de la figure 2. On retrouve dans ce schéma le générateur formé des éléments 1 à 8, le connecteur 9, la paire d'électrodes 10 et la résistance 11. L'instrument 12 a été supprimé.

Comme le potentiomètre 2 est gradué, cet instrument est remplacé par une diode émettrice de lumière (LED) 14 dont l'intensité lumineuse varie proportionnellement à la densité de courant. A cet effet, l'entrée qui inverse de l'amplificateur 6 est reliée à l'entrée directe d'un amplificateur opérationnel 13 avec un circuit de contre-réaction formé d'une diode LED 14 et d'une résistance 15. Le courant dans la diode 14 est égal à $U_s/R15$, c'est-à-dire à $K \cdot U_{smax}/R15$, intensité proportionnelle à la position du curseur du potentiomètre 2, au moins dans les conditions normales de fonctionnement.

Une des premières anomalies qui peut se produire est une résistance trop élevée au passage du courant dans les électrodes. Cette résistance trop élevée peut être due au fait que les électrodes sont mal mises en place et qu'elles sont partiellement décollées, ce qui produit une diminution de la surface effective de passage du courant et une surintensité locale. Il est donc nécessaire de limiter la tension aux bornes des électrodes. Cette tension $U_e$ est proportionnelle à la résistance $R_e$ entre électrodes:

$$U_e = I_e \, R_e$$

Lorsque la résistance $R_e$ augmente, la tension sur l'émetteur du transistor 8 augmente jusqu'au moment où elle s'approche de la tension d'alimentation. A ce moment, le système se sature et le courant dans les électrodes atteint une valeur limite qu'il ne peut plus dépasser. Par un choix judicieux entre la tension d'alimentation et la tension $U_{smax}$, on obtient donc une limitation automatique du courant dans les électrodes lorsque la résistance $R_e$ augmente de manière critique. Cette situation anormale doit toutefois être signalée. A cet effet, l'émetteur du transistor 8 est relié à l'entrée directe d'un comparateur de tension 18 dont l'entrée qui inverse est branchée à un diviseur de tension formé des résistances 16 et 17 délivrant sur cette entrée qui inverse une tension de référence légèrement inférieure à la tension d'alimentation.

Lorsque l'on s'approche de la saturation du système, la tension sur l'émetteur du transistor 8 dépasse la tension de référence et la sortie du comparateur 18 devient +V, rendant conductrice une diode émettrice de lumière (LED) 19 utilisée comme diode de signalisation, le courant dans cette diode étant limité par une résistance 20 branchée contre la masse.

On obtient ainsi une indication parfaitement claire d'une augmentation anormale de la résistance entre les électrodes, grâce à l'allumage de la diode 19 d'indication de saturation.

Nous avons vu que lorsque le système est saturé, le courant Ie ne peut être maintenu à la valeur fixée. La valeur de la tension sur la résistance 11 devient donc inférieure à la tension Us de consigne.

Lorsque la saturation est faible, il n'y a pas lieu de s'alarmer et l'utilisateur peut se contenter de d'amener le potentiomètre 2 dans une position inférieure dans laquelle le système ne sature plus.

Par contre, lorsque la saturation est très forte, c'est-à-dire lorsque la tension aux bornes de la résistance 11 devient très inférieure à la tension de consigne, il y a lieu d'interrompre immédiatement le circuit pour éviter tout risque de brûlure lors du traîtement.

Pour celà, on dispose d'un circuit de sécurité comportant un comparateur de tension 21 dont l'entrée qui inverse est reliée à l'entrée qui inverse de l'amplificateur 6 et par la borne 9b du connecteur 9 à la résistance 11, l'entrée directe de ce comparateur 21 étant branchée à l'entrée qui inverse de l'amplificateur 3. La tension sur cette entrée est déterminée par le diviseur de tension formé des résistances R4 et R5 selon la relation:

$$US \quad \frac{R5}{R5 + R4}$$

Lorsque la tension aux bornes de R11 tombe en dessous de cette valeur, la sortie du comparateur 21 passe à +V. Un courant circule alors dans une diode émettrice de lumière 22 (LED) de signalisation de "sécurité", puis par un optocoupleur 23 dans la base d'un transistor 24 dont l'émetteur est à la masse et dont le collecteur est reliée à la base du transistor 8.
Ce transistor 24 devient donc conducteur et il court-circuite la base du transistor 8 contre la masse. Le transistor 8 devient non conducteur et il ne passe plus aucun courant dans les électrodes. L'alimentation en courant est interrompue.

Ainsi, lorsque les conditions de fonctionnement deviennent par trop anormales ou critiques le système de sécurité est immédiatement déclenché, ce qui se traduit par l'interruption du courant et par l'allumage d'une diode de signalisation "sécurité".

Les sorties de l'optocoupleur 23 peuvent également être reliées aux entrées d'une sirène 30 qui délivre un signal accoustique afin de prévenir l'opérateur si celui-ci n'est pas à proximité de l'appareil lorsque la sécurité se déclenche.

Pour remettre le circuit en fonction, l'opérateur doit remettre le potentiomètre 2 à zéro. A ce moment l'entrée directe du comparateur 21 passe à zéro alors que l'entrée qui inverse est maintenue à un potentiel légèrement positif par la résistance 25 de valeur élevée, de sorte que la sortie du comparateur 21 passe à zéro ce qui éteint la diode 22, déclenche l'optocoupleur 23 et rend non conducteur le transistor 24.

Enfin, bien que le bon fonctionnement du circuit soit indiqué pas les différentes diodes LED de signalisation, il peut être utile de vérifier de manière simple le courant qui passe dans les électrodes, et ceci sans interrompre le circuit ce qui aurait pour effet de déclencher la securité. Dans ce but, une résistance de faible valeur 26 est connecté en série dans le circuit d'alimentation des électrodes. Cette résistance 26 est reliée aux deux bornes d'un connecteur 27, connecteur sur lequel on peut brancher un instrument de mesure numérique ou analogique 28.

Les appareils d'ionisation comportent toujours plusieurs paires d'électrodes et plusieurs générateurs de courant, il est ainsi possible de vérifier successivement le courant dans chaque paire d'électrodes au moyen d'un seul instrument de mesure en branchant celui-ci sur les connecteurs prévus à cet effet.

La figure 3 montre un exemple de disposition possible des éléments 2,14,19 et 22, permettant un contrôle rapide du bon fonctionnement du dispositif. Un potentiomètre linéaire associé à une graduation en mA/cm$^2$ permet un dosage précis du courant pour tous les types de paires d'électrodes utilisés.

0092015

- 1 -

<u>REVENDICATIONS</u>

1. Dispositif d'ionisation comportant au moins un générateur de courant réglable (3,6) et au moins une paire d'électrodes (10) branchée de manière amovible audit générateur par des moyens de connexion (9), caractérisé
par le fait qu'il comprend en outre des moyens (11) reliés audit générateur (3,6) pour déterminer l'intensité du courant délivré par ledit générateur auxdites électrodes (10a,10b), lesdits moyens (11) dépendant du type de la paire d'électrodes (10) branchée audit générateur.

2. Dispositif selon la revendication 1, caractérisé
par le fait que ledit générateur de courant comprend un premier amplificateur opérationnel (3) relié à un potentiomètre (2) et comportant un circuit de contre-réaction (4,5) et
un second amplificateur opérationnel (6) relié au premier amplificateur opérationnel (3), avec un circuit de contre-réaction comprenant d'une part un premier transistor (8) et ladite paire d'électrodes (10) et d'autre part lesdits moyens (11) déterminant l'intensité du courant dans lesdites électrodes (10a,10b).

3. Dispositif selon les revendications 1 ou 2,

- 2 -                     0092015

caractérisé

par le fait que lesdits moyens (11) sont solidaires de ladite paire d'électrodes (10) et reliés à une fiche de branchement de ladite paire d'électrodes afin d'être branchés audit générateur (3,6) par lesdits moyens de connexion (9) losque ladite paire d'électrodes est connectée audit générateur.

4. Dispositif selon les revendications 1 ou 2, caractérisé

par le fait que lesdits moyens sont une résistance (11) dont la valeur dépend de la surface (S) desdites électrodes (10a,10b) de ladite paire, afin de maintenir constante la densité de courant dans lesdites électrodes.

5. Dispositif selon la revendication 3, caractérisé

par le fait que la valeur de ladite résistance (11) est déterminée par la surface de la plus petite desdites électrodes (10a,10b) de ladite paire.

6. Dispositif selon la revendication 2, caractérisé

par le fait que la densité de courant dans les électrodes (10a,10b) est mesurée par un instrument (12) gradué en mA/cm$^2$ et branché à la sortie dudit premier amplificateur opérationnel (3).

7. Dispositif selon la revendication 2, caractérisé

par le fait que ledit potentiomètre de réglage (2) comporte une échelle graduée en mA/cm$^2$ devant laquelle se déplace le curseur dudit potentiomètre.

8. Dispositif selon la revendication 7, caractérisé par le fait qu'il comporte un circuit (13) branché auxdits moyens (11) et alimentant une diode (LED) émettrice de lumière (14) avec un courant dont l'intensité est proportionnelle à la position du curseur dudit potentiomètre (2), de sorte que l'intensité lumineuse de la dite diode (14) varie proportionnellement à la densité de courant dans lesdites électrodes (10a,10b).

9. Dispositif selon la revendication 2, caractérisé par le fait qu'il comporte un premier circuit de comparaison (16,17,18) pour comparer la tension de sortie dudit premier transistor (8) à une tension de référence, ledit circuit de comparaison commandant une diode (LED) émettrice de lumière (19) pour signaler une saturation dudit transistor (8) produite par l'augmentation de ladite tension de sortie lorsque la résistance entre les électrodes (10a,10b) dépasse une valeur critique déterminée, ladite saturation donnant lieu à une limitation automatique du courant dans les électrodes.

10. Dispositif selon les revendications 2 ou 9, caractérisé par le fait qu'il comprend un second circuit de comparaison (21) pour comparer la tension aux bornes desdits moyens (117 à la tension d'entrée du premier amplificateur opérationnel (37 et commander une diode (LED) émettrice de lumière (22) dite de signalisation de sécurité, branchée en série avec un second transistor (24) connecté en parallèle sur l'entrée dudit premier transistor (8) pour court-circuiter cette entrée et bloquer le premier transistor (8), de manière à interrompre le courant dans les électrodes (10a,10b) lorsque, en raison d'une augmentation de la résistance entre les électrodes, la ten-

- 4 -

0092015

sion aux bornes desdits moyens (11) tombe en dessous de ladite tension d'entrée.

11. Dispositif selon la revendication 10, caractérisé par le fait que ladite diode de signalisation de sécurité (22) est couplée audit second transistor (24) par un optocoupleur (23) commandant une sirène délivrant un signal acoustique lorsque ledit premier transistor (8) ne délivre plus de courant dans lesdites électrodes (10a,10b).

12. Dispositif selon la revendication 1, caractérisé par le fait qu'il comprend des moyens (26) branchés en série avec les dites électrodes et un dispositif d'affichage (28) susceptible d'être relié auxdits moyens pour mesurer le courant dans lesdites électrodes (10a,10b) sans interrompre le circuit.

Fig 1

Fig 3

Contrôle.

15    22

mA/Cm²

0,10
0,09
0,08
0,07
0,06
0,05
0,04
0,03
0,02
0,01
0,00

14

sécurité

2/1

0092015

Fig.2

Office européen
des brevets

RAPPORT DE RECHERCHE EUROPEENNE

0092015
Numéro de la demande

EP 82 81 0160

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 180 666 (SYBRON)<br><br>*Page 3, lignes 13-18; page 4, lignes 5-32, page 5, lignes 8-26; page 6, lignes 5-27* | 1,3,4, 9 | A 61 N 1/30<br>A 61 N 1/08 |
| A | CH-A- 385 366 (ROSE)<br>*Page 2, lignes 7-12* | 1 | |
| A | FR-A-2 153 191 (CHATEAU DE CRESSIA)<br>*Page 3, ligne 21 - page 4, ligne 12* | 2,6 | |
| A | US-A-4 141 359 (JACOBSEN)<br>*Colonne 6, ligne 12 - colonne 8, ligne 6* | 2,9,10 | |
| | | | **DOMAINES TECHNIQUES RECHERCHES (Int. Cl. 3)** |
| A | GB-A-2 064 178 (SYBRON)<br><br>*Page 2, lignes 10-21,37-45,112-117; page 3, lignes 3-6* | 6,8-10 ,12 | A 61 N |

Le présent rapport de recherche a été établi pour toutes les revendications

| Lieu de la recherche | Date d'achèvement de la recherche | Examinateur |
|---|---|---|
| LA HAYE | 25-11-1982 | SIMON J.J.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................
& : membre de la même famille, document correspondant

OEB Form 1503. 03.82